# EUROPÄISCHE PATENTSCHRIFT

(11) **EP 3 372 176 B1**
(45) Veröffentlichungstag und Bekanntmachung des Hinweises auf die Patenterteilung: **04.09.2019**
(21) Anmeldenummer: 18160451.3
(22) Anmeldetag: 07.03.2018
(51) Int. Cl.: A61B 17/28, A61B 17/04, A61B 17/062, A61B 17/29, A61B 17/00

(54) **MEDIZINISCHES INSTRUMENT**
MEDICAL INSTRUMENT
INSTRUMENT MÉDICAL

(30) Priorität: 10.03.2017 DE 102017204010
(43) Veröffentlichungstag der Anmeldung: 12.09.2018
(73) Patentinhaber: Richard Wolf GmbH, 75438 Knittlingen (DE)
(72) Erfinder: Amann, Carina, 75449 Wurmberg (DE); Hähnle, Friedrich, 75015 Bretten (DE); Körner, Eberhard, 75438 Knittlingen (DE)
(74) Vertreter: Patentanwälte Vollmann Hemmer Lindfeld Partnerschaft mbB

(56) Entgegenhaltungen:
- WO-A1-2015/168441
- DE-A1-102012 211 200
- DE-A1-102014 110 881
- JP-A- H07 275 253

## Beschreibung

Die Erfindung betrifft ein medizinisches Instrument, insbesondere ein endoskopisches Instrument mit den im Oberbegriff des Anspruchs 1 angegebenen Merkmalen.

Ein medizinisches Instrument dieser Art ist aus DE 42 07 124 C1 bekannt. Es handelt sich hierbei um ein medizinisches Instrument wie beispielsweise eine Klemme, eine Zange, einen Nadelhalter oder dergleichen, welches proximalseitig eine Handhabe zum Steuern eines distalseitig angeordneten Werkzeuges aufweist, wobei die Handhabe zwei relativ zueinander schwenkbeweglich angeordnete und federkraftbeaufschlagte Griffstücke aufweist. Diese Griffstücke sind über eine zwischen den Griffstücken angeordnete Bahnführung miteinander bewegungsgekoppelt, wobei die Bahnführung aus einem eine Führungsbahn aufweisenden Bahnbauteil, das an einem Griffstück angelenkt ist, und einem am anderen Griffstück angeordneten einen Führungskörper aufweisenden Führungsbauteil besteht, wobei der Führungskörper mit der Führungsbahn in Eingriff steht. Dabei sind die Griffstücke zueinander federkraftbeaufschlagt, darüber hinaus ist der Führungskörper in Richtung zum Grund der Führungsbahn federkraftbeaufschlagt, wodurch die Umlaufrichtung des Führungskörpers in der Führungsbahn zumindest abschnittsweise bestimmt ist. Innerhalb der Führungsbahn sind mehrere Raststellungen vorgesehen, in welche der Führungskörper eingreifen kann, sodass beim Zusammendrücken der Griffstücke diese in einer der Raststellungen verrasten können, wobei bei weiterem Zusammendrücken der Griffstücke die jeweilige Raststellung gelöst und die Griffstücke in die nächste Raststellung oder wieder federkraftbedingt auseinanderbewegt werden. Diese Raststellungen sind dazu vorgesehen, um das distale Werkzeug, beispielsweise einen Nadelhalter in der Haltefunktion zu fixieren und nachfolgend wieder zu lösen.

Nachteilig bei dieser bekannten Konstruktion ist, dass aufgrund der offenen Anordnung nahe den proximalen Enden der Griffstücke die Gefahr besteht, dass sich ein Fremdkörper in die Rastmechanik setzt und deren sichere Funktion behindert. Um die notwendige Federkraft aufzubringen sowie die Haltekraftfunktion zu gewährleisten, müssen die Bauteile zwischen den Griffstücken ausreichend stabil ausgebildet sein, was das Instrument schwer und unhandlich macht.

Eine ähnliche Rastmechanik ist aus DE 10 2014 110 881 A1 bekannt, auch hier ist ein Führungskörper mit einer Führungsbahn im Eingriff, wobei der Führungskörper nicht nur die Führungsfunktion innerhalb der Führungsbahn übernimmt sondern auch die Haltekraft in der Raststellung, sodass eine Miniaturisierung der Bauteile schon aufgrund der zu übertragenden Kräfte kaum mehr möglich ist.

Vor diesem Hintergrund liegt der Erfindung die Aufgabe zugrunde, ein gattungsgemäßes medizinisches Instrument zu verbessern, insbesondere so auszubilden, dass die Rastmechanik weiter miniaturisiert werden kann ohne deren Funktion zu beeinträchtigen.

Diese Aufgabe wird gemäß der Erfindung durch ein medizinisches Instrument mit den in Anspruch 1 angegebenen Merkmalen gelöst. Vorteilhafte Ausgestaltungen der Erfindung sind in den Unteransprüchen, der nachfolgenden Beschreibung und den Zeichnungen angegeben.

Das erfindungsgemäße medizinische Instrument ist insbesondere ein endoskopisches Instrument, also ein Instrument mit einer proximalen Handhabe zum Steuern eines distalseitigen Werkzeugs, die typischerweise über einen Schaft und eine darin geführte Mechanik, z. B. eine Stange miteinander verbunden und bewegungsgekoppelt sind. Die proximale Handhabe weist zwei relativ zueinander schwenkbeweglich angeordnete Griffstücke auf, die federkraftbeaufschlagt sind und die über eine Bahnführung miteinander bewegungsgekoppelt sind. Die Bahnführung weist ein eine Führungsbahn aufweisendes Bahnbauteil und ein in die Führungsbahn eingreifenden Führungskörper an einem Führungsbauteil auf. Diese Bauteile sind jeweils an einem der Griffstücke angeordnet und zueinander quer zu einer Schwenkebene der Griffstücke federkraftbeaufschlagt. Eines dieser Bauteile ist schwenkbar an einem Griffstück angeordnet, wobei zwischen diesen Bauteilen mindestens eine Raststellung gebildet ist, in welcher die Schwenkbewegung der Griffstücke in einer Richtung blockiert ist.

Gemäß der Erfindung ist funktional unabhängig von Führungskörper und Führungsbahn ein Rastkörper für die Rastfunktion vorgesehen sowie mindestens eine den Rastkörper in einer Raststellung aufnehmende Rastaufnahme, und zwar an den Bauteilen, d. h. am Führungsbauteil und am Bahnbauteil.

Grundgedanke der erfindungsgemäßen Lösung ist somit die Entkopplung von Führungs- und Rastfunktion. Dadurch, dass für die Rastfunktion ein vom Führungskörper und Führungsbahn funktional unabhängiger Rastkörper sowie eine dafür vorgesehene Rastaufnahme vorgesehen sind, können die Bauteile den jeweiligen Funktionen entsprechend angepasst werden, d. h. lediglich der Rastkörper und die Rastaufnahme sind zur Aufnahme vergleichsweise größere Haltekräfte zu dimensionieren, die entstehen, wenn die Griffstücke in geschlossener Stellung gehalten werden, d. h. das Werkzeug, z. B. der Nadelhalter in Schließstellung gehalten wird. Führungskörper und Führungsbahn hingegen können auf die reinen Führungsfunktionen während des Bewegungsablaufs, d. h. beim Schwenken der Griffstücke zueinander bzw. beim federkraftbedingten Rückschwenken dimensioniert werden, womit eine erhebliche Miniaturisierung einhergehen kann, die es ermöglicht, diese Bauteile wesentlich weiter distalwärts, also in der Nähe des oder der Gelenkverbindungen der Griffstücke anzuordnen. Hierdurch sind die Griffstücke besser zugänglich und weniger anfällig für Fehlbedienungen oder Fehlfunktionen durch Eindringen von Fremdkörpern.

Dabei spielt es gemäß der Erfindung grundsätzlich keine Rolle, ob ein Griffstück instrumentenfest und das andere schwenkbar angeordnet ist oder ob beide Griffstücke bezogen auf einen Instrumentenkörper schwenkbar angeordnet sind. Auch spielt es für die erfindungsgemäße Lösung keine Rolle, ob die Federkraftbeaufschlagung der Griffstücke in Öffnungs- oder in Schließrichtung vorgesehen ist, gleichwohl diese typischerweise in Öffnungsrichtung wirksam ist, d. h. beim Aufeinanderzuschwenken der Griffstücke diese Federkraft überwunden werden muss, welche in Gegenrichtung dafür sorgt, dass die Griffstücke einschließlich des damit verbundenen Werkzeuges öffnen.

Grundgedanke der Erfindung ist es somit, die Führungsfunktion und die Rastfunktion voneinander zu entkoppeln, d. h. funktional unabhängig voneinander konstruktiv zu gestalten. Hierdurch können insbesondere Führungskörper und Führungsbahn kleiner dimensioniert werden, was vorteilhaft ist. Typischerweise kann der Führungskörper als schlanker Stift konzipiert werden wohingegen der Rastkörper mit der Rastaufnahme zur Aufnahme der Haltekräfte des Instrumentes größer und stabiler dimensioniert wird. Grundsätzlich wird die Rastfunktion so ausgelegt werden, dass eine Raststellung vorgesehen ist, welche die Griffstücke in ihrer Schließstellung hält. Gemäß der Erfindung können jedoch auch mehrere Raststellungen vorgesehen sein, für Zwischenstellungen, auch kann eine Raststellung in der geöffneten Stellung der Griffstücke vorgesehen sein.

Dabei kann gemäß der Erfindung der Rastkörper am Bahnbauteil und die mindestens eine Rastaufnahme am Führungsbauteil angeordnet sein oder auch umgekehrt. Die Anordnung des Rastkörpers am Bahnbauteil kann konstruktiv günstig sein, wenn sich diese in Verbindung mit der Bahnführung schon aus Platzgründen ergibt.

Die Ausbildung von Rastkörper und Rastaufnahme können vielfältig sein, insbesondere können diese an die konstruktiven Gegebenheiten angepasst sein. Besonders vorteilhaft ist es, wenn bei der Ausgestaltung, bei der der Rastkörper am Bahnbauteil und die Rastaufnahme am Führungsbauteil angeordnet sind, der Rastkörper durch einen zapfenartigen Vorsprung gebildet ist, dessen Achse quer zu einer Schwenkebene der Griffstücke angeordnet ist, wobei der Zapfen in Richtung zum Führungsbauteil gerichtet ist und die Rastaufnahme durch einen Vorsprung ebenfalls quer zu einer Schwenkebene der Griffstücke gebildet ist, jedoch Richtung zum Bahnbauteil. Dann stehen diese beiden Teile, nämlich der Rastkörper einerseits und die Rastaufnahme andererseits so zueinander, dass sie mit ihren Umfangsflächen in Raststellung in Eingriff kommen. Diese Bauteile sind dann hinsichtlich ihrer Querschnittsflächen entsprechend den aufzunehmenden Kräften zu dimensionieren.

Alternativ können gemäß einer vorteilhaften Weiterbildung der Erfindung der Rastkörper am Führungsbauteil angeordnet und die mindestens eine Rastaufnahme am Bahnbauteil angeordnet sein. Auch bei dieser Konstellation wird der Rastkörper vorteilhaft durch einen zapfenartigen Vorsprung gebildet, der quer zu einer Schwenkebene der Griffstücke angeordnet und zum Bahnbauteil hin gerichtet ist. Dann ist die Rastaufnahme durch einen Vorsprung quer zu einer Schwenkebene aber in Gegenrichtung, d. h. in Richtung zum Führungsbauteil gebildet. Rastkörper und Vorsprung erheben sich also jeweils quer zu Schwenkebenen der Griffstücke, jedoch in entgegengesetzte Richtung.

Unter Schwenkebene der Griffstücke ist eine Ebene zu verstehen, in welcher die Griffstücke beim Aufeinanderzubewegen bzw. beim Voneinanderwegbewegen liegen. Da es sich bei den Griffstücken um sich räumlich erstreckende Bauteile handelt, ergeben sich eine Vielzahl solcher gedachten Ebenen. Grundsätzlich kann es sich hierbei auch um Ebenen parallel dazu handeln.

Bei dieser Konstellation, bei welcher der Rastkörper am Führungsbauteil und die Rastaufnahme am Bahnbauteil angeordnet sind, ist es besonders vorteilhaft, wenn der Führungskörper am Ende des Rastkörpers angeordnet ist und einen kleineren Querschnitt als der Rastkörper aufweist. Rast- und Führungskörper sind dann vorzugsweise achsgleich durch einen abgestuften Zapfen gebildet, wobei der am Führungsbauteil angeordnete im Querschnitt größere Zapfenabschnitt den Rastkörper und der sich davon erstreckende im Querschnitt kleinere Zapfenabschnitt den Führungskörper bildet.

Besonders vorteilhaft ist es, wenn neben der Rastaufnahme mindestens eine Führungsfläche für den Rastkörper vorgesehen ist, welcher den Rastkörper in eine Richtung quer zu einer Schwenkebene der Griffstücke unmittelbar vor und/oder nach Erreichen der Raststellung steuert. Auf diese Weise wird der Führungskörper stirnseitig von der Führungsbahn entkoppelt, sodass dieser in der Tat nur noch die reine Bahnführungsfunktion erfüllen muss wohingegen die mindestens eine Führungsfläche neben der Rastaufnahme die Steuerung des Rastkörpers in Richtung der Zapfenachse - wenn dieser als Zapfen ausgebildet ist - übernimmt.

Grundsätzlich spielt es für die Funktion keine Rolle, ob das Führungsbauteil oder das Bahnbauteil schwenkbar an einem Griffstück gelagert ist, besonders vorteilhaft ist es jedoch, das Führungsbauteil schwenkbar am Griffstück zu lagern, da dieses bevorzugt als schmales, flaches Bauteil mit dem Führungskörper nahe dem freien Ende vorgesehen ist, welches bevorzugt nach Art einer Blattfeder ausgebildet ist und in Richtung quer zu einer Schwenkebene der Griffstücke in Richtung zum Bahnbauteil vorgespannt ausgebildet ist. Die Federvorspannung sorgt dafür, dass der Führungskörper stets mit dem Boden der Führungsbahn Kontakt hat und so durch die Führungsbahn in Querrichtung zur Schwenkebene der Griffstücke gesteuert wird. Bei einer solchen Anordnung ist es besonders vorteilhaft, wenn das Führungsbauteil in Richtung quer zu einer Schwenkebene der Griffstücke und zwar in Gegenrichtung zum Bahnbauteil, also auf der vom Bahnbauteil abgewandten Seite durch einen Abschnitt der Handhabe bzw. das Griffstück abgedeckt ist. Eine solche Anordnung sorgt dafür, dass das Führungsbauteil zumindest an einer Seite abgedeckt und somit geschützt ist, wobei dieser Schutz nicht nur das Bauteil selbst sondern auch den Bediener vor diesem Bauteil schützt, insbesondere verhindert, dass Fremdkörper in diesen Bereich eindringen. Zur anderen Seite ist das Führungsbauteil durch das Bahnbauteil zumindest teilweise abgedeckt und geschützt.

Um die gezielte Bahnsteuerung in nur eine Richtung auch in den Bereich zu gewährleisten, in welchem der Führungskörper nicht mehr mit dem Boden der Bahnführung in Eingriff steht, ist es vorteilhaft, mindestens zwei oder mehr Führungsflächen neben der Rastaufnahme so anzuordnen, dass zwischen ihnen eine Stufe gebildet ist. Diese Stufe, die dann für die Bewegung des Rastkörpers wirksam ist, verhindert, dass sich der Rastkörper auf seiner Bahn zum Eingriff mit der Rastaufnahme bzw. aus dieser heraus in eine andere als die vorbestimmte Bahnrichtung bewegt.

Gemäß einer vorteilhaften Weiterbildung der Erfindung ist die Führungsbahn nutförmig ausgebildet, liegt also typischerweise in einem flächigen Bauteil, wie beispielsweise ein Blech, in welches die Führungsbahn eingefräst oder in anderer Weise ausgebildet ist, wobei der Nutgrund Schrägflächen und Stufen aufweist, die zur Bildung von Rücklaufsperren vorgesehen sind. Die Seiten dieser Nut bilden die eigentlichen Führungsflächen der Führungsbahn. Der Nutgrund verhindert durch die Abstufungen, dass der Führungskörper, der mit seiner Stirnseite am Nutgrund anliegt und dort entlanggleitet in der Führungsbahn rückwärts bewegt wird, also sich entgegen der vorbestimmten Bewegungsrichtung bewegt. Dabei sind Schrägflächen vorgesehen, welche den Anstieg zu einer Stufe überbrücken. Beim Auflaufen einer solchen Schrägfläche wird der Führungskörper angehoben bis er nach Erreichen der Stufe wieder abgesenkt wird, sodass eine Rückbewegung durch die senkrechte Kante der Stufe, deren senkrechte Fläche, parallel zum Außenumfang des Führungszapfens liegt, wenn der Führungskörper als Zapfen ausgebildet ist, einen Rücklauf verhindert.

Vorteilhaft weist gemäß der Erfindung die Führungsbahn einen umlaufenden Bahnabschnitt auf, in dem zwei gleich gerichtete Umlenkbereiche vorgesehen sind, in denen eine Umlenkung der Bahn von mehr als 90° und weniger als 180° erfolgt wobei ein dazwischen liegender entgegengerichteter Umlenkbereich angeordnet ist.

Unter Umlenkbereich im Sinne der vorliegenden Erfindung ist ein Bahnabschnitt zu verstehen, in welchem eine Umlenkung d. h. Richtungsänderung erfolgt. Bei gleich gerichteten Umlenkbereichen erfolgt die Umlenkung in gleiche Richtung, d. h. in bestimmungsgemäßem Verlauf der Bahnführung gesehen entweder in Form einer Umlenkung also einer Kurve nach rechts oder in Form einer Umlenkung also einer Kurve nach links. Unter entgegen gerichteter Umlenkung ist zu verstehen, dass diese Umlenkung, wenn die davor angeordnete Umlenkung nach rechts umlenkt diese entgegen gerichtete Umlenkung nunmehr nach links umlenkt und umgekehrt.

Vorteilhaft schließt gemäß einer Weiterbildung der Erfindung der umlaufende Bahnabschnitt an einen Bahnabschnitt an, der sowohl beim Auseinanderschwenken als auch beim Zueinanderschwenken der Griffstücke eine Führungsbahn bildet. Ein solcher Bereich liegt außerhalb der eigentlichen Rastmechanik und dient zur Führung in beide Bewegungsrichtungen, ermöglicht es also insbesondere, dass die Griffstücke auch über die übliche Handhabung hinaus gespreizt werden können, dies jedoch funktional unabhängig von der Rastmechanik, wie dies beispielsweise zum Anschließen von Schaft und Steuerstange bei endoskopischen Instrumenten erforderlich sein kann.

In dem umlaufenden Bahnabschnitt sind vorteilhaft gemäß einer Weiterbildung der Erfindung die Rücklaufsperren so angeordnet und ausgerichtet, dass die Führungsbahn nur in eine Richtung durchfahrbar ist, also eine definierte Bewegungsabfolge erfolgt.

Gemäß einer vorteilhaften Weiterbildung der Erfindung kann bei dem medizinischen Instrument die Rastfunktion abschaltbar sein, indem beispielsweise mittels eines einschwenkbaren Hebels oder verschiebbaren Körpers das Führungsbauteil seitlich, d. h. in Richtung quer zu einer Schwenkebene der Griffstücke bewegt wird, sodass sowohl Führungskörper als auch Rastkörper außer Eingriff mit der Führungsbahn bzw. der Rastaufnahme gelangen. Dies kann insbesondere bei Ausgestaltung des Führungsbauteils nach Art einer Blattfeder auf einfache Weise dadurch erfolgen, dass das Führungsbauteil entgegen seiner Federvorspannung angestellt wird. Hierzu genügt es an der Innenseite des entsprechenden Griffstücks einen Schieber oder Hebel anzubringen, welcher das Führungsbauteil gegenüber dem Bahnbauteil beispielsweise durch Einschieben eines Keils voneinander beabstandet.

Die Erfindung ist nachfolgend anhand von in den Zeichnungen dargestellten Ausführungsbeispielen näher erläutert. Es zeigen:
- Fig. 1: in stark vereinfachter durchsichtiger Ansicht den proximalen Teil eines endoskopischen Nadelhalters,
- Fig. 2: in vergrößerter Darstellung die Rastmechanik des Instrumentes gemäß Fig. 1,
- Fig. 3: in perspektivischer Draufsicht das Bahnbauteil des Instrumentes gemäß Fig. 1,
- Fig. 4: in perspektivischer Draufsicht das Führungsbauteil des Instrumentes nach Fig. 1,
- Fig. 5: in vergrößerter perspektivischer Draufsicht den die Führungsbahn bildenden Teil eines Bahnbauteils einer alternativen Ausführungsvariante,
- Fig.6: in perspektivischer Draufsicht das mit dem Bahnbauteil gemäß Fig. 5 in Eingriff bringbare Führungsbauteil dieser Ausführung,
- Fig.7: eine dritte Ausführung in Darstellung nach Fig. 5 und
- Fig.8: das zugehörige Führungsbauteil in Darstellung nach Fig. 6.

Bei dem anhand der Figuren dargestellten Instrument handelt es sich um einen endoskopischen Nadelhalter 1, dessen grundsätzlicher Aufbau zum Stand der Technik zählt. Das eigentliche Werkzeug, der Nadelhalter ist am distalen Ende eines Schaftes 2 angeordnet, der in einer proximalen Handhabe 3 aufgenommen ist. Das Werkzeug ist über eine im Schaft 2 geführte Steuerstange betätigbar, die Betätigung erfolgt mittels der Handhabe 3.

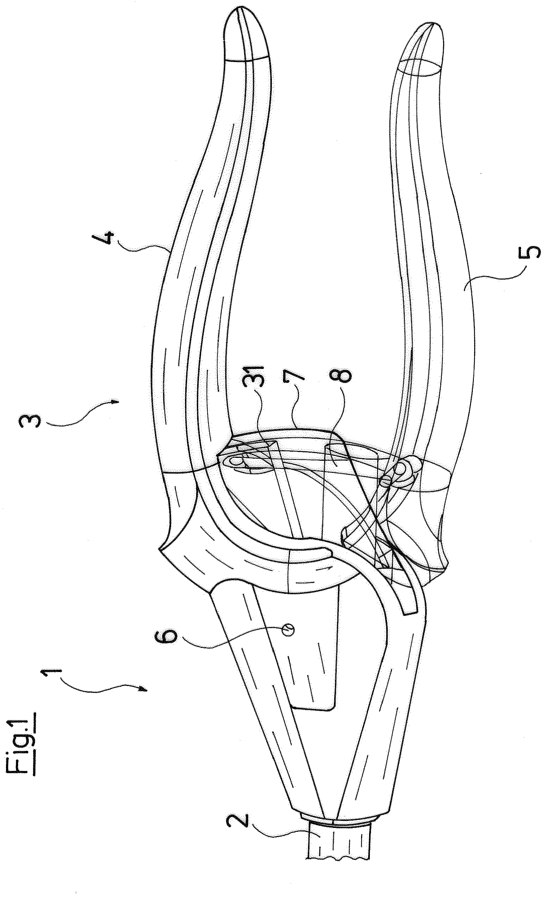

Bei der dargestellten Ausführungsform weist die Handhabe 3 ein starr mit dieser verbundenes Griffstück 4 sowie ein daran schwenkbeweglich gelagertes Griffstück 5 auf, welches um eine Drehachse 6 schwenkbar ist. Das Griffstück 5 ist gegenüber dem Griffstück 4 in Öffnungsrichtung federkraftbelastet, hierzu ist innerhalb der Handhabe 3 um die Drehachse 6 eine Schraubentorsionsfeder eingegliedert, deren Enden einerseits am Griffstück 4 und andererseits am Griffstück 5 abgestützt sind, sodass die Griffstücke entgegen der in Fig. 1 dargestellten geschlossenen Stellung federvorgespannt sind. In dieser geschlossenen Stellung ist auch das Werkzeug geschlossen, d. h. eine mit dem Nadelhalter gegriffene Nadel festgehalten.

Die Handhabe 3 verrastet in dieser Fig. 1 dargestellten geschlossenen Stellung, wenn die Griffstücke 4 und 5 typischerweise durch eine diese umfassende Hand soweit wie möglich zusammengedrückt und dann losgelassen werden. Erst beim erneutem Zusammendrücken und nachfolgendem Loslassen schwenkt das Griffstück 5 durch die Kraft der vorgespannten Feder gegenüber dem Griffstück 4 um die Drehachse 6 soweit aus, bis der Abstand der proximalen Enden der Griffstücke 4 und 5 maximal ist.

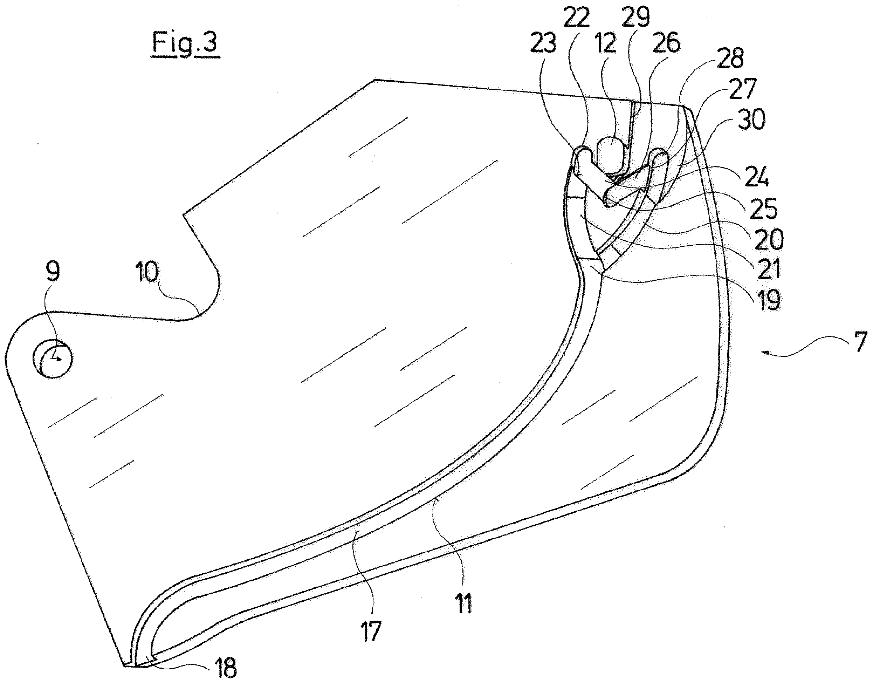

Die Rastmechanik ist zwischen den Griffstücken 4 und 5 ausgebildet und weist ein Bahnbauteil 7 sowie ein damit in Eingriff stehendes Führungsbauteil 8 auf. Das Bahnbauteil 7, welches in Fig. 3 im Einzelnen dargestellt ist, ist am Griffstück 4 formschlüssig festgelegt, zum einen durch einen die Drehachse 6 bildenden Bolzen, welcher eine Bohrung 9 durchsetzt und zum anderen durch ein Formschlussmittel, welches in eine Randausnehmung 10 eingreift und zusammen mit der Festlegung an der Bohrung 9 dafür sorgt, dass das Bahnbauteil 7 fest und starr mit dem Griffstück 4 verbunden ist. Das Bahnbauteil 7 ist plattenförmig und kann beispielsweise durch ein Edelstahlblech gebildet sein, es weist eine Führungsbahn 11 auf, die weiter unten noch im Einzelnen beschrieben ist sowie einen Rastkörper in Form eines Zapfens 12, welcher sich gegenüber der in Fig. 3 sichtbaren Oberfläche (aus der Papierebene) heraus erhebt.

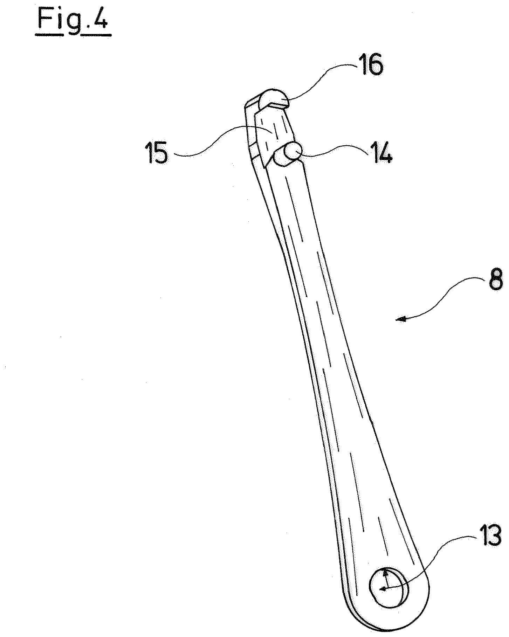

Das Bahnbauteil 7 steht mit dem Führungsbauteil 8 in Wirkverbindung, welches in Fig. 4 dargestellt ist. Die in Fig. 4 sichtbare Flachseite des Führungsbauteils 8 ist in Einbaulage der in Fig. 3 sichtbaren Flachseite des Bahnbauteils 7 zugewandt. Das Führungsbauteil 8 ist ebenfalls als flächiges Bauteil ausgebildet, und zwar nach Art einer Blattfeder. Es weist an seinem einen Ende eine Bohrung 13 auf, mit der es schwenkbar an der Innenseite des Griffstücks 5 festgelegt ist, wie dies anhand von Fig. 1 ersichtlich ist. Nahe dem freien Ende des Führungsbauteils 8 ist ein Führungskörper in Form eines Führungszapfens 14 angeordnet, der sich von dem flächigen Bauteil 8 senkrecht erhebt und eine zylindrische Form aufweist. Dieser Zapfen 14 erstreckt sich senkrecht zu einer gedachsten Ebene, welche beim Schwenken des Griffstücks 5 um die Achse 6 gegenüber dem Griffstück 4 aufgespannt wird.

Zum freien Ende des Führungsbauteils 8 schließt sich an den Zapfen 14 eine zurückspringende Fläche 15 an, die eine Führungsfläche bildet und am Ende von einer parallel zum Zapfen 12 vorspringenden Rastaufnahme 16 abgeschlossen wird. Diese Rastaufnahme 16 hat die Querschnittsform einer Mondsichel, deren nach außen gerichtete Wölbung durch die abgerundete Form des freien Endes des Führungsbauteils 8 gebildet ist und deren eingezogene Innenseite dem Zapfen 12 gegenüberliegt. Im Übrigen ist die Form des Führungsbauteils 8 von seinem griffstückseitigen Ende nahe der Bohrung 13 bis zum freien Ende insbesondere im letzten Drittel mit zunehmender Materialstärke jedoch abnehmender Breite ausgebildet, sodass das griffstückseitige Ende eher Federfunktion übernimmt, wohingegen das freie Ende eher als starr betrachtet werden kann.

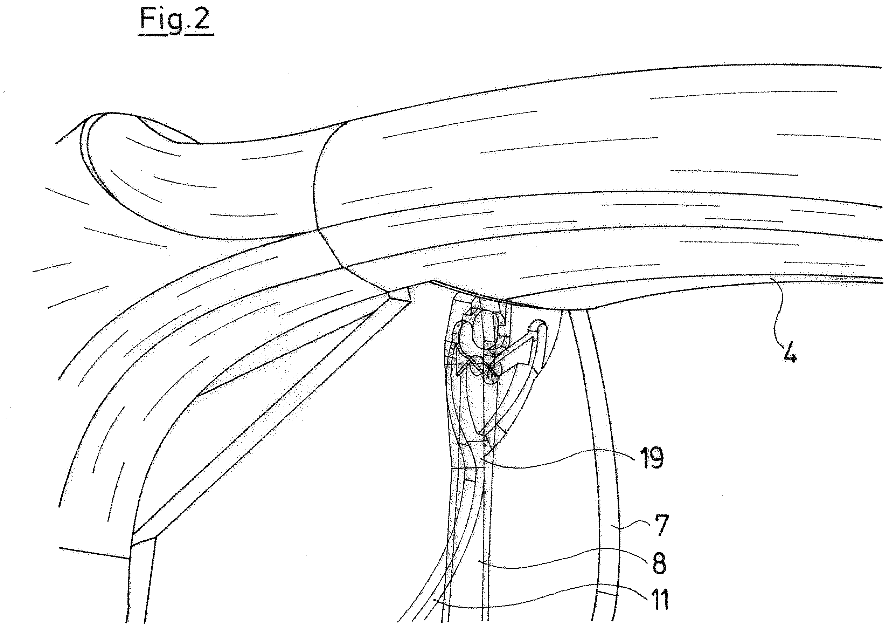

Der Führungszapfen 14 springt deutlich weiter hervor als der die Rastaufnahme 16 bildende Vorsprung, sodass in Einbaulage (siehe Fig. 1 und 2) der Zapfen 14 in die Führungsbahn 11 eingreift, welche den Zapfen 14 seitlich führt. Durch die Federkraft des blattfederartigen ausgebildeten Führungsbauteils 8 wird der Führungszapfen 14 mit seiner Stirnseite auf den Grund 17 der Führungsbahn 11 gedrückt. Dieser Grund 17 verläuft eben und parallel der Bauteiloberfläche von einer seitlichen Eingangsöffnung 8 bis zum Beginn 19 eines umlaufenden Führungsbahnabschnitts. Dieser umlaufende Führungsbahnabschnitt weist zwei Schrägflächen 20, 21 auf, nämlich die in Fig. 3 rechte Schrägfläche 20, die vom Beginn des umlaufenden Führungsbahnabschnitts 19 schräg nach unten, d. h. in die Papierebene der Fig. 3 hinein auf ein tieferes Niveau führt und eine in Fig. 3 linke Schrägfläche 21, welche von einem Niveau 17 der Führungsbahn 11 weiter nach oben führt.

Beim Aufeinanderzubewegen der Griffstücke 4 und 5 wird der Führungszapfen 14 längs der Führungsbahn 11 zum Beginn des umlaufenden Führungsbahnabschnitts 19 bewegt. Aufgrund seiner Anordnung wirkt auf den Zapfen 14 eine bezogen auf Fig. 3 nach links wirkende Kraft, die dazu führt, dass beim weiteren Zusammendrücken der Griffstücke 4 und 5 der Zapfen 14 in den in Fig. 3 linken Zweig des umlaufenden Führungsabschnitts gelangt um dort zunächst über die Schrägfläche 21 auf ein höheres Niveau gebracht zu werden. Wenn die Griffstücke 4 und 5 maximal zusammengedrückt sind, dann befindet sich der Führungszapfen 14 am Ende des in Fig. 3 linken Teils des umlaufenden Führungsbahnabschnitts, der einen Umlenkbereich 22 bildet. Hier macht die Führungsbahn in bestimmungsgemäßer Durchlaufrichtung gesehen eine Rechtskurve. Werden die Griffstücke 4 und 5 bei Erreichen dieser Endstellung kraftentlastet, so werden diese durch Federkraft wieder ein kleines Stück auseinanderbewegt, wobei aufgrund der überwundenen Stufe 23 der Führungszapfen 14 nicht zurück in den Bereich der Schrägfläche 21 laufen kann, sondern einen kurzen Bahnbereich 24 durchläuft bis der Zapfen 14 eine Stufe 25 überfährt in welcher eine Raststellung gebildet ist, die weiter unten noch im Einzelnen beschrieben ist. Bei nochmaligen Zusammendrücken der Griffstücke 4 und 5 durchfährt der Führungszapfen 14 den zweiten kurzen Bahnbereich 26 und gelangt schließlich über eine Stufe 27 in einen Umlenkbereich 28, der durch die Stufe 27 von dem kurzen Bahnbereich 26 getrennt ist. Werden nun die Griffstücke 4 und 5 erneut kraftentlastet, so wandert der Zapfen 14, da er aufgrund der Stufe 27 nicht in den kurzen Bahnbereich 26 zurück kann, über die Schrägfläche 20 wieder zum Anfang 19 des umlaufenden Führungsbahnabschnitts und von dort auf den langgestreckten Teil der Führungsbahn 11. Der Führungszapfen 14 macht also in Durchlaufrichtung gesehen zunächst im Umlenkbereich 22 eine Rechtskurve, durchfährt dann einen entgegen gerichteten Umlenkbereich im Bereich der Stufe 25, macht also eine Linkskurve um dann im Umlenkbereich 28 erneut eine Rechtskurve zu durchlaufen.

Bei der beschriebenen Ausführung dient der in der Führungsbahn 11 laufende Führungszapfen 14 ausschließlich der Bahnführung und nicht der Rastfunktion. Letztere wird durch den Rastkörper 12 in Verbindung mit der Rastaufnahme 16 erreicht, wobei insbesondere von Bedeutung ist, dass die Haltekräfte für die Rastfunktion durch diese beiden Bauteile 12, 16 und nicht durch den Führungszapfen 14 und die Führungsbahn aufgenommen werden, der in der Rastposition insoweit lastfrei ist. Der Rastzapfen 12 läuft mit geringen Spiel außerhalb der Führungsbahn 11 neben dem Führungszapfen 14 der Art, dass beim Durchfahren des Führungszapfens 14 durch den umlaufenden Führungsbahnabschnitt vom Beginn 19 bis zum Umlenkbereich 22 die Rastaufnahme 16 so verfährt, dass der Rastzapfen 12 zwischen der Rastaufnahme 16 und dem Führungszapfen 14 angeordnet ist. Fährt nun der Führungszapfen 14 den kurzen Bahnbereich 24 bis jenseits der Stufe 25 entlang, gelangt die eingezogene Innenseite der Rastaufnahme 16 zur Anlage an dem Rastzapfen 12. In dieser Raststellung ist der Führungszapfen 14 kraftentlastet. Dabei sorgt die zurückspringende Fläche 15 dafür, die an der Stirnseite des Rastzapfens 12 anliegt auch für eine Kraftentlastung des Führungszapfens 14 in Achsrichtung. Beim erneuten Zusammendrücken der Griffstücke 4 und 5 und Durchfahren des kurzen Bahnbereichs 26 wird dann die Rastaufnahme 16 über eine Stufe 29 bewegt, an die sich eine Schrägfläche 30 anschließt, die in der Oberfläche des Bahnbauteils 7 mündet.

Wie sich aus den schematischen Darstellungen nach Fig. 1 entnehmen lässt, ist der Rastmechanismus innerhalb der Handhabe 3 zur einen Seite durch die glatte Außenseite des Bahnbauteils 7 und zur anderen Seite durch eine Schutzabdeckung 31 abgedeckt, sodass dieser Bereich von außen praktisch nicht zugänglich ist, sodass sowohl die Rastfunktion als auch die Führungsfunktion im Bereich der umlaufenden Führungsbahn vollständig geschützt liegen.

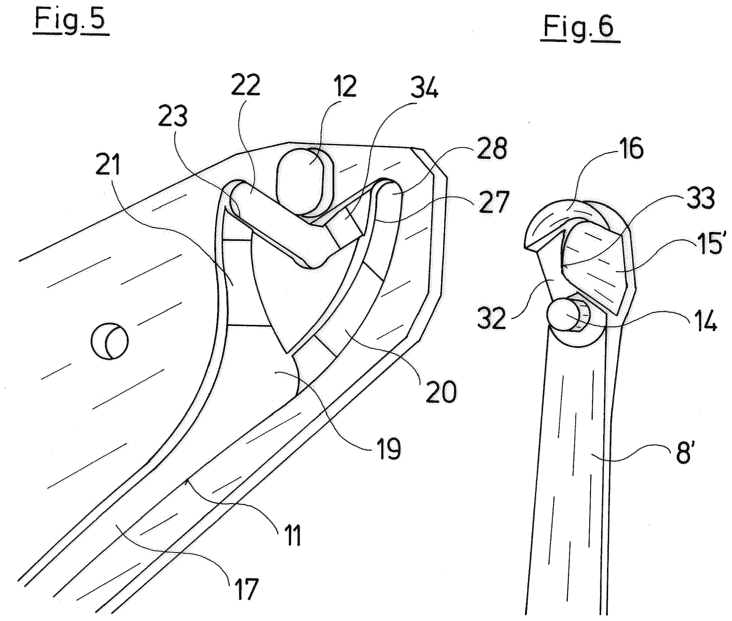

Bei der anhand der Fig. 5 und 6 dargestellten Ausführungsvariante sind ein Bahnbauteil 7' und ein Führungsbauteil 8' vorgesehen, die sich von der vorbeschriebenen Ausführungsvariante dadurch unterscheidet, dass in dem Bahnbauteil 7' die Stufe 29 und die Schrägfläche 30 des Bahnbauteils 7 fehlen, d. h. diese Bereiche können bei der Ausführung gemäß in Bahnbauteil 7' unbearbeitet bleiben, jedoch ist innerhalb der Führungsbahn 11 die Stufe 25 durch eine Schrägfläche 34 ersetzt. Entsprechend dazu weist das Führungsbauteil 8' keine durchgängig zurückspringende Fläche 15 sondern eine Fläche 15' auf, an die sich über eine Stufe 33 eine niveauhöhere Fläche 32 anschließt. Die Flächen 32 und 15' bilden Führungsflächen und werden wirksam, sobald diese Bereiche über die Stirnfläche des Rastzapfens 12 gelangen. Dabei ersetzt die Stufe 33 des Führungsbauteils 8' die Stufe 29 im Bahnbauteil 7. Diese sorgt also dafür, dass unter Entlastung des Führungszapfens 14 der Rastzapfen 12 nach Überwinden der Stufe 33 an der zurückspringenden Fläche 15' anliegend in seine definierte Stellung in der Rastaufnahme 16 gelangt, aus der wegen der Stufe 33 nur in Richtung des Führungszapfens 14 durch den kurzen Bahnbereich 26 zum Umlenkbereich 28 verfahren werden kann. Dabei sorgt die Schrägfläche 34 dafür, dass der Führungszapfen 14 wieder auf ein höheres Niveau geführt wird um dann die eine Rückwärtsbewegung sperrende Stufe 27 herabzufallen.

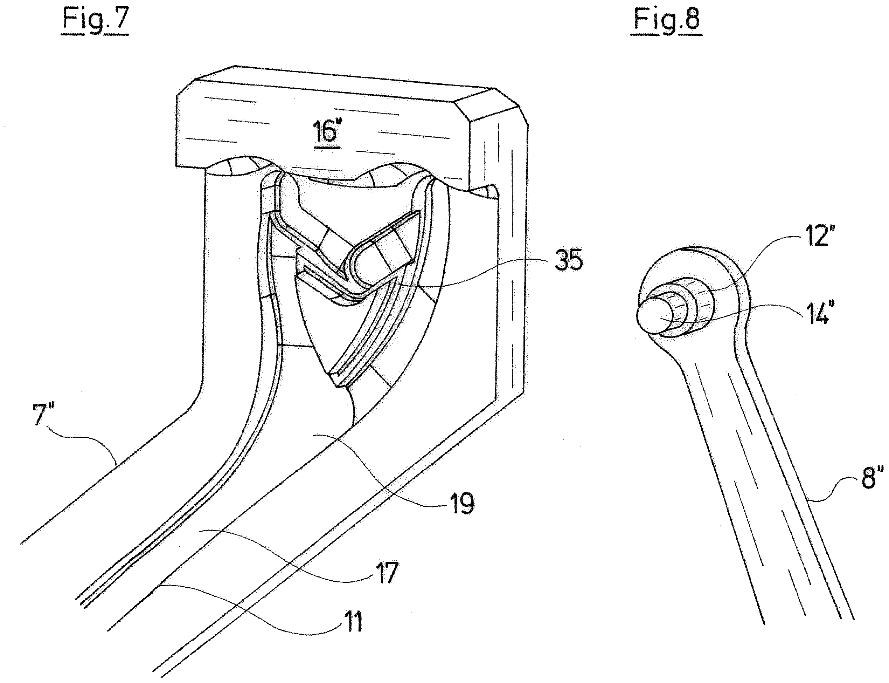

Anhand der Fig. 7 und 8 sind Ausführungsvarianten eines Bahnbauteils 7" und eines Führungsbauteils 8" gezeigt, die sich von den vorstehend beschriebenen Ausführungsformen dadurch unterscheiden, dass sowohl der Rastzapfen 12" als auch der Führungszapfen 14", der stirnseitig an dem Rastzapfen 12" achsgleich anschließt, am Führungsbauteil 8" angeordnet ist, wohingegen die Rastaufnahme 16" am Bahnbauteil 7" angeordnet ist. Bei dieser Ausführungsvariante sind ebenfalls Führungs- und Rastfunktion voneinander getrennt, allerdings setzt der Führungszapfen 14" den Rastzapfen 12" mit geringerem Querschnitt fort. Entsprechend sind im umlaufenden Führungsbahnabschnitt Führungsflächen 35 vorgesehen, in denen die stirnseitige Abstufung des Rastzapfens 12" die axiale Führung übernimmt und der Zapfen 12" die Kräfte in Verbindung mit Rastaufnahme 16" in der Rastposition aufnimmt.

### Bezugszeichenliste

- 1 -: endoskopischer Nadelhalter
- 2 -: Schaft
- 3 -: Handhabe
- 4 -: starres Griffstück
- 5 -: bewegliches Griffstück
- 6 -: Drehachse/Bolzen
- 7, 7', 7" -: Bahnbauteil
- 8, 8, 8" -: Führungsbauteil
- 9 -: Bohrung
- 10 -: Randausnehmung
- 11 -: Führungsbahn
- 12, 12" -: Rastkörper/Rastzapfen
- 13 -: Bohrung im Führungsbauteil
- 14 -: Führungskörper/Führungszapfen
- 15, 15' -: zurückspringende Fläche
- 16, 16" -: Rastaufnahme
- 17 -: Grund der Führungsbahn
- 18 -: Eingangsöffnung der Führungsbahn
- 19 -: Beginn des umlaufenden Führungsbahnabschnitts
- 20 -: rechte Schrägfläche
- 21 -: linke Schrägfläche
- 22 -: linker Umlenkbereich
- 23 -: Stufe
- 24 -: kurzer Bahnbereich
- 25 -: Stufe
- 26 -: kurzer Bahnbereich
- 27 -: Stufe
- 28 -: rechter Umlenkbereich
- 29 -: Stufe
- 30 -: Schrägfläche (Führungsfläche)
- 31 -: Schutzabdeckung
- 32 -: Führungsfläche
- 33 -: Stufe
- 34 -: Schrägfläche
- 35 -: Führungsfläche

## Patentansprüche

1. Medizinisches Instrument, insbesondere endoskopisches Instrument (1), mit einer proximalen Handhabe (3) zum Steuern eines distalen Werkzeugs, bei dem die Handhabe (3) zwei relativ zueinander schwenkbeweglich angeordnete und federkraftbeaufschlagte Griffstücke (4, 5) aufweist, die über eine Bahnführung (11, 14) miteinander bewegungsgekoppelt sind, wobei die Bahnführung (11, 14) ein eine Führungsbahn (11) aufweisendes Bahnbauteil (7) und ein in die Führungsbahn (11) eingreifenden Führungskörper (14) an einem Führungsbauteil (8) aufweist, wobei die Bauteile (7, 8) an den Griffstücken (4, 5) angeordnet und zueinander quer zu einer Schwenkebene der Griffstücke (4, 5) federkraftbeaufschlagt sind, eines der Bauteile (8) schwenkbar an dem Griffstück (5) angeordnet ist und mindestens eine Raststellung zwischen den Bauteilen (7, 8) gebildet ist, in welcher die Schwenkbewegung der Griffstücke (4, 5) in eine Richtung blockiert ist, **dadurch gekennzeichnet, dass** funktional unabhängig von Führungskörper (14) und Führungsbahn (11) für die Rastfunktion ein Rastkörper (12) und mindestens eine den Rastkörper (12) in einer Raststellung aufnehmende Rastaufnahme (16) an den Bauteilen (7, 8) vorgesehen sind.

2. Medizinisches Instrument nach Anspruch 1, **dadurch gekennzeichnet, dass** der Rastkörper (12) am Bahnbauteil (7) und die mindestens eine Rastaufnahme (16) am Führungsbauteil (8) angeordnet ist.

3. Medizinisches Instrument nach Anspruch 2, **dadurch gekennzeichnet, dass** der Rastkörper (12) durch einen zapfenartigen Vorsprung (12) quer zu einer Schwenkebene der Griffstücke (4, 5) in Richtung zum Führungsbauteil (8) und die Rastaufnahme (16) durch einen Vorsprung (16) quer zu einer Schwenkebene der Griffstücke (4, 5) in Richtung zum Bahnbauteil (7) gebildet ist.

4. Medizinisches Instrument nach Anspruch 1, **dadurch gekennzeichnet, dass** der Rastkörper (12") am Führungsbauteil (8") und die mindestens eine Rastaufnahme (16") am Bahnbauteil (7") angeordnet ist.

5. Medizinisches Instrument nach Anspruch 4, **dadurch gekennzeichnet, dass** der Rastkörper (12) durch einen zapfenartigen Vorsprung quer zu einer Schwenkebene der Griffstücke (4, 5) in Richtung zum Bahnbauteil (7") gebildet ist und die Rastaufnahme (16") durch einen Vorsprung quer zu einer Schwenkebene der Griffstücke (4, 5) in Richtung zum Führungsbauteil (7") gebildet ist.

6. Medizinisches Instrument nach Anspruch 5, **dadurch gekennzeichnet, dass** der Führungskörper (14") am Ende des Rastkörpers (12") angeordnet ist und einen kleineren Querschnitt als der Rastkörper (12") aufweist.

7. Medizinisches Instrument nach einem der vorhergehenden Ansprüche, **dadurch gekennzeichnet, dass** vorzugsweise neben der Rastaufnahme (16) mindestens eine Führungsfläche (15, 15', 32) für den Rastkörper (12) vorgesehen ist, welche den Rastkörper (12) in einer Richtung quer zu einer Schwenkebene der Griffstücke (4, 5) unmittelbar vor und nach Erreichen der Raststellung steuert.

8. Medizinisches Instrument nach einem der vorhergehenden Ansprüche, **dadurch gekennzeichnet, dass** das Führungsbauteil (8) schwenkbar an dem Griffstück (5) gelagert ist, dass das Führungs bauteil (8) nach Art einer Blattfeder ausgebildet und in Richtung quer zu einer Schwenkebene der Griffstücke (4, 5) in Richtung zum Bahnbauteil (7) vorgespannt ausgebildet ist, und/oder dass das Führungsbauteil (8) in Richtung quer zu einer Schwenkebene der Griffstücke (4, 5) in Gegenrichtung zum Bahnbauteil (7) durch einen Abschnitt (31) der Handhabe (3) abgedeckt ist.

9. Medizinisches Instrument nach einem der vorhergehenden Ansprüche, **dadurch gekennzeichnet, dass** mindestens zwei Führungsflächen (15', 32) neben der Rastaufnahme (16) angeordnet sind, zwischen denen eine Stufe (33) gebildet ist.

10. Medizinisches Instrument nach einem der vorhergehenden Ansprüche, **dadurch gekennzeichnet, dass** die Führungsbahn (11) nutförmig ausgebildet ist, wobei der Nutgrund (17) Schrägflächen (20, 21, 34) aufweist, die zur Bildung von Rücklaufsperren (23, 25, 27, 29) zueinander abgestuft sind, dass die Führungsbahn (11) einen umlaufenden Bahnabschnitt aufweist, in dem zwei gleich gerichtete Umlenkbereiche (22, 28) vorgesehen sind, in denen eine Umlenkung der Bahn von mehr als 90° und weniger als 180° erfolgt, und ein dazwischen liegender entgegen gerichteter Umlenkbereich angeordnet ist, dass der umlaufende Bahnabschnitt an einen Bahnabschnitt anschließt, der sowohl beim Auseinanderschwenken als auch beim Zueinanderschwenken der Griffstücke eine Führungsbahn (11) bildet, und/oder dass Rücklaufsperren (23, 25, 27, 29) in einem umlaufenden Bahnabschnitt so ausgerichtet und angeordnet sind, dass dieser nur in einer Richtung durchfahrbar ist.

11. Medizinisches Instrument nach einem der vorangehenden Ansprüche, **dadurch gekennzeichnet, dass** die Rastfunktion deaktivierbar ist.

## Claims

1. A medical instrument, in particular endoscopic instrument (1), with a proximal handle (3) for controlling a distal tool, concerning which the handle (3) comprises two grip pieces (4, 5) which are arranged in a pivotably movable manner relative to one another, are subjected to spring force and are coupled in movement to one another via a path guide (11, 14), wherein the path guide (11, 14) comprises a path component (7) which comprises a guide path (11), and a guide body (14) on a guide component (8), said guide body engaging into the guide path (11), wherein the components (7, 8) are arranged on the grip pieces (4, 5) and are subjected to spring force to one another transversely to a pivoting plane of the grip pieces (4, 5), one of the components (8) is pivotably arranged on a grip piece (5) and at least one latching position, in which the pivoting movement of the grip pieces (4, 5) is blocked in one direction, is formed between the components (7, 8), **characterised in that** in a manner which is functionally independent of the guide body (14) and guide path (11), a latching body (12) and at least one latching receiver (16) which receives the latching body (12) in a latching position are provided on the components (7, 8) for the latching function.

2. A medical instrument according to claim 1, **characterised in that** the latching body (12) is arranged on the path component (7) and the at least one latching receiver (16) on the guide component (8).

3. A medical instrument according to claim 2, **characterised in that** the latching body (12) is formed by a stub-like projection (12) transverse to a pivoting plane of the grip pieces (4, 5) in the direction of the guide component (8) and the latching receiver (16) is formed by a projection (16) transverse to a pivoting plane of the grip pieces (4, 5) in the direction of the path component (7).

4. A medical instrument according to claim 1, **characterised in that** the latching body (12") is arranged on the guide component (8") and the at least one latching receiver (16") is arranged on the path component (7").

5. A medical instrument according to claim 4, **characterised in that** the latching body (12) is formed by a stub-like projection transverse to a pivoting plane of the grip pieces (4, 5) in the direction of the path component (7") and the latching receiver (16") is formed by a projection transversely to a pivoting plane of the grip pieces (4, 5) in the direction of the guide component (7").

6. A medical instrument according to claim 5, **characterised in that** the guide body (14") is arranged at the end of the latching body (12") and has a smaller cross section than the latching body (12").

7. A medical instrument according to one of the preceding claims, **characterised in that** preferably, next to the latching receiver (16), at least one guide surface (15, 15', 32) is provided for the latching body (12), said guide surface guiding the latching body (12) in a direction transverse to a pivoting plane of the grip pieces (4, 5) directly before and after reaching the latching position.

8. A medical instrument according to one of the preceding claims, **characterised in that** the guide component (8) is pivotably mounted on the grip piece (5), that the guide component (8) is designed in the manner of a leaf spring and is designed biased in the direction of the path component (7) in the direction transverse to a pivoting plane of the grip pieces (4, 5) and/or that the guide component (8) is covered by a section (31) of the handle (3) in a direction transverse to a pivoting plane of the grip pieces (4, 5) in the direction opposite to the path component (7).

9. A medical instrument according to one of the preceding claims, **characterised in that** at least two guide surfaces (15', 32) are arranged next to the latching receiver (16), between which a step (33) is formed.

10. A medical instrument according to one of the preceding claims, **characterised in that** the guide path (11) is designed in a groove-like manner, wherein the groove base (17) comprises oblique surfaces (20, 21, 34) which are stepped to one another for forming return stops (23, 25, 27, 29), that the guide path (11) comprises a peripheral path section, in which two equally directed deflecting regions (22, 28) are provided, in which a deflection of the path of more than 90° and less than 180° is effected, and an oppositely directed deflecting region is arranged lying therebetween, that the peripheral path section connects to a path section which forms a guide path (11) on pivoting the grip pieces apart as well as pivoting them together, and/or that return stops (23, 25, 27, 29) are aligned and arranged in a peripheral path section such that this can only be travelled through in one direction.

11. A medical instrument according to one of the preceding claims, **characterised in that** the latching function can be deactivated.

## Revendications

1. Instrument médical, notamment instrument endoscopique (1), avec une poignée (3) proximale pour commander un outil distal, dans lequel la poignée (3) comprend deux pièces de poignée (4, 5) aptes à pivoter l'une par rapport à l'autre et précontraintes par un effort de ressort, lesquelles pièces étant accouplées en mouvement, l'une à l'autre, par le biais d'un guidage de trajet (11, 14), le guidage de trajet (11, 14) comprenant un composant de trajet (7) comportant une piste de trajet (11) et, sur un composant de guidage (8), un corps de guidage (14) s'engageant dans la piste de trajet (11), les composants (7, 8) étant disposés sur les pièces de poignée (4, 5) et étant précontraints par un effort de ressort, l'un par rapport à l'autre, transversalement à un plan de pivotement des pièces de poignée (4, 5), un des composants étant disposé pivotant sur la pièce de poignée (5) et au moins une position d'encliquetage étant formée entre les composants (7, 8) dans laquelle le mouvement de pivotement des pièces de poignée (4, 5) est bloquée dans une direction, **caractérisé en ce que** sont prévus sur les composants (7, 8), de façon fonctionnellement indépendante du corps de guidage (14) et de la piste de trajet (11), un corps d'encliquetage (12) pour la fonction d'encliquetage et au moins un logement d'encliquetage (16) pour recevoir le corps d'encliquetage (12) dans une position d'encliquetage.

2. Instrument médical selon la revendication 1, **caractérisé en ce que** le corps d'encliquetage (12) est disposé sur le composant de trajet (7) et ledit au moins un logement d'encliquetage (16) est disposé sur le composant de guidage (8).

3. Instrument médical selon la revendication 2, **caractérisé en ce que** le corps d'encliquetage (12) est constitué par une avancée (12) en forme de tenon transversalement à un plan de pivotement des pièces de poignées (4, 5) en direction du composant de guidage (8), et que le logement d'encliquetage (16) est constitué par une avancée transversalement à un plan de pivotement des pièces de poignées (4, 5) en direction du composant de trajet (7).

4. Instrument médical selon la revendication 1, **caractérisé en ce que** le corps d'encliquetage (12") est fixé au composant de guidage (8") et ledit au moins un logement d'encliquetage (16") est fixé au composant de trajet (7").

5. Instrument médical selon la revendication 4, **caractérisé en ce que** le corps d'encliquetage (12) est constitué par une avancée en forme de tenon transversalement à un plan de pivotement des pièces de poignées (4, 5) en direction du composant de trajet (7") et que le logement d'encliquetage (16") est constitué par une avancée transversalement à un plan de pivotement des pièces de poignées (4, 5) en direction du composant de guidage (7").

6. Instrument médical selon la revendication 5, **caractérisé en ce que** le corps de guidage (14") est disposé à l'extrémité du corps d'encliquetage (12") et présente une coupe transversale plus petite que le corps d'encliquetage (12").

7. Instrument médical selon une des revendications précédentes **caractérisé en ce que**, de préférence à côté du logement d'encliquetage (16), est prévu pour le corps d'encliquetage (12) au moins une surface de guidage (15, 15', 32) qui commande le corps d'encliquetage (12) dans une direction transversalement à un plan de pivotement des pièces de poignées (4, 5) immédiatement avant et après avoir atteint la position d'encliquetage.

8. Instrument médical selon une des revendications précédentes, **caractérisé en ce que** le composant de guidage (8) est logé pivotant sur la pièce de poignée (5), que le composant de guidage (8) est formé à la façon d'un ressort à lames et est formé en direction transversalement à un plan de pivotement des pièces de poignées (4, 5) et de manière précontrainte en direction du composant de trajet (7), et/ou que le composant de guidage (8) est recouvert, dans la direction transversale à un plan de pivotement des pièces de poignées (4,5), par un segment (31) de la poignée (3) dans la direction inverse par rapport au composant de trajet (7).

9. Instrument médical selon l'une des revendications précédentes, **caractérisé en ce qu'**au moins deux surfaces de guidage (15', 32) sont disposées à côté du logement d'encliquetage (16) entre lesquelles est formé un étage (33).

10. Instrument médical selon l'une des revendications précédentes, **caractérisé en ce que** la piste de trajet (11) présente une forme rainurée, le fond de rainure (17) comprenant des surfaces obliques (20, 21, 34) qui sont étagées les unes par rapport aux autres pour constituer des blocages anti-retour (23, 25, 27, 29), que la piste de trajet (11) comprend un segment de trajet périphérique dans lequel sont prévus deux zones de renvoi (22, 28) orientées dans le même sens dans lesquelles se produit un renvoi du trajet de plus de 90° et de moins de 180°, et dans lesquelles est intercalée une zone de renvoi orientée en sens inverse, que le segment de trajet périphérique est adjacent à un segment de trajet qui forme une piste de trajet (11) aussi bien par le pivotement d'éloignement que par le pivotement de rapprochement des pièces de poignées, et/ou que, dans un segment de trajet périphérique, des blocages anti-retour (23, 25, 27, 29) sont orientés et disposés de telle façon que celui-ci ne puisse être traversé que dans une direction.

11. Instrument médical selon l'une des revendications précédentes, **caractérisé en ce que** la fonction d'encliquetage est désactivable.
